# EUROPÄISCHE PATENTANMELDUNG

(11) **EP 2 394 574 A1**
(43) Veröffentlichungstag der Anmeldung: **14.12.2011**
(21) Anmeldenummer: 10165638.7
(22) Anmeldetag: 11.06.2010
(51) Int. Cl.: A61B 5/11

(54) **System und Verfahren zum Analysieren und/oder Trainieren eines Sprach-, Schluck- und/oder Atemvorgangs**

(71) Anmelder: Ratiopharm GmbH, 89079 Ulm (DE)
(72) Erfinder: Heim, Bernhard, 89079, Ulm (DE)
(74) Vertreter: TER MEER - STEINMEISTER & PARTNER GbR

(57) **Zusammenfassung**

Die Erfindung betrifft ein System und ein Verfahren zum Analysieren und/oder Trainieren eines Sprach-, Schluck- und/oder Atemvorgangs, wobei dreidimensionale optische und ortsaufgelöste akustische Daten erfasst werden und ein Sprech-, Schluck- und/oder Atemvorgang basierend auf den akustischen und optischen Daten von einer Kontrolleinheit analysiert wird.

## Beschreibung

Die Erfindung betrifft ein System und ein Verfahren zum Analysieren und/oder Trainieren eines Sprach-, Schluck- und/oder Atemvorgangs.

Die Anwendung von Inhalatoren stellt für ältere oder kranke Benutzer oft ein Problem dar. Oft wird das Mundstück nicht ausreichend fest mit den Lippen umschlossen oder nicht tief genug eingeatmet, sodass das Wirkstoffe enthaltende Inhalationsvolumen nicht an einen Wirkungsort, z. B. in die Bronchien, gelangt. Auch erfordert insbesondere die Verwendung von nicht-Atemzug-gesteuerten Inhalatoren eine sorgfältige Koordination des Aerosolausstoßes mit dem Einatmungsvorgang. Ein wiederholtes Üben und Vorführen eines optimalen Inhalationsvorgangs durch geschultes Personal ist allerdings teuer und aufwändig.

Bei Sprachentwicklungsstörungen, neurologischen Insulten und neurodegenerativen Erkrankungen kommt es ferner durch eine Sprach-, Sprech-, Stimm- oder Schluckbeeinträchtigung zu einer Einschränkung der zwischenmenschlichen Kommunikationsfähigkeit des Betroffenen. Eine therapeutische Behandlung beispielsweise durch einen Logopäden ist oftmals nicht möglich, da eine solche Behandlung eine große finanzielle und unter Umständen auch gesundheitliche Belastung darstellt. Insbesondere für ältere und gebrechliche Patienten, wie beispielsweise Parkinsonpatienten, kann eine Anfahrt zum Logopäden zu umständlich und gefährlich sein. Allerdings konnte bei Parkinsonpatienten, die aufgrund ihrer Erkrankung an einer zu leisen Stimme, einer verwaschenen Aussprache und einer monotonen Sprechweise leiden, durch logopädische Therapie eine starke Verbesserung der Mundbeweglichkeit, der Mimik, des Schluckens und der Atmung festgestellt werden.

Aus diesen Gründen wurden kürzlich Computerprogramme entwickelt, die logopädisches Training zu Hause ermöglichen. Diese computergestützten Therapien haben allerdings den Nachteil, dass nur der akustische Erfolg einer Übung bewertet wird. Es fehlt also sowohl an einer optischen Erfolgskontrolle, die die Mimik und Bewegung von Gesichts- und Halsmuskulatur berücksichtigt, als auch an einer visuellen Vorgabe durch einen logopädischen Therapeuten. Häufig lassen sich jedoch logopädische Übungen besser durch Vorführen zeigen als beschreiben. Außerdem können mit den herkömmlichen Systeme kleine Fortschritte nicht festgestellt werden, die sich eventuell nur in der Verwendung von bestimmten Gesichtsmuskeln zeigen und akustisch nicht erkennbar sind.

Daher ist es Aufgabe der vorliegenden Erfindung, ein System und ein Verfahren zum Analysieren und/oder Trainieren eines Sprach-, Schluck- und/oder Atemvorgangs bereit zu stellen, das eine günstige logopädische Therapierung oder ein Applikationstraining von Inhalatoren ermöglicht, das einen Sprech-, Atmungs- oder Inhalationsvorgang sowohl optisch als auch akustisch erfassen bzw. einen optimalen Vorgang vorführen und auch kleinste Fortschritte durch eine Totalerfassung sämtlicher für ein Training relevanter Parameter feststellen kann und das an eine individuelle Lernkurve anpassbar ist.

Diese Aufgabe wird durch die Merkmale der unabhängigen Ansprüche gelöst.

Die Erfindung basiert auf dem Gedanken, ein 3D-Kamerasystem mit einem akustischen Kamerasystem zu kombinieren, um ein Sprach-, Schluck- oder Atemtraining mittels einer optischen und akustischen Echtzeit-3D-Erfolgskontrolle zu beurteilen. Hierfür weist ein erfindungsgemäßes System für Sprach-, Schluck- und/oder Atemtraining eine optische Einheit zur dreidimensionalen optischen Datenerfassung eines Vorgangs auf. Ein Vorgang bezeichnet beispielsweise Prozesse während einer Sprech-, Atem-, Schluck- oder Inhalationsübung. Die optische Einheit ist vorzugsweise dazu geeignet, eine Körperoberfläche eines Benutzers dreidimensional und zeitaufgelöst zu erfassen. Darüber hinaus umfasst das erfindungsgemäße System eine akustische Einheit, die während des Vorgangs erzeugten Schall ortsaufgelöst detektieren kann. Vorzugsweise werden die akustischen und optischen dreidimensionalen Daten gleichzeitig bzw. simultan erfasst. Darüber hinaus kann die akustische Einheit einen Entstehungsort von Schall ermitteln und den detektierten Schall jeweils bezüglich Entstehungsort, Frequenz, Amplitude und/oder Dauer auflösen. Des Weiteren weist das System eine Kontrolleinheit auf, die den Vorgang basierend auf den erfassten akustischen und optischen Daten analysiert. Die Kontrolleinheit kann beispielsweise einen Computer umfassen. Die Analyse der erfassten Daten beruht vorzugsweise auf einem Vergleich mit Vorgabedaten, die im System vorhanden sind. Zur Beurteilung des Trainings können entweder Absolutwerte der Vorgabedaten oder auch Relativwerte herangezogen werden, wobei die aktuell erfassten Daten mit Daten von früheren Trainingseinheiten verglichen werden. Dabei können einzelne Parameter, die für die jeweilige Übung wichtig sind, z.B. für Tiefe der Atmung, Stimmhöhe, Lautstärke, Tondauer, etc., unabhängig voneinander ausgewertet werden. Die Auswertung bzw. die Analyseergebnisse und mögliche Trainingsanweisungen werden von der Kontrolleinheit an eine Ausgabeeinheit weitergeleitet.

Vorzugsweise kann das System in Echtzeit einen Vorgang erfassen und umgehend Feedback an einen Benutzer geben. Hierfür ist die optische Einheit vorzugsweise für eine dreidimensionale Echtzeit-Bildaufnahme ausgelegt. In einem besonders bevorzugten Ausführungsbeispiel umfasst die optische Einheit mindestens eine Time-Of-Flight-Kamera (TOF-Kamera). In einem Ausführungsbeispiel kann die optische Einheit neben einem allgemeinen Gesichtsausdruck bzw. einer Mund- oder Lippenstellung auch eine Bewegung oder Anspannung von Gesichtsmuskeln erfassen, sodass eine genauere Analyse beispielsweise des Sprechvorgangs möglich wird. Vorzugsweise kann die optische Einheit Bewegungen im Gesicht-, Hals- und/oder Thoraxbereich des Benutzers erfassen, um der Kontrolleinheit und/oder einer Ausgabeeinheit optisch erfasste Daten eines Vorgangs bereitzustellen. Wenn mehrere Kameras verwendet werden, können auch Bereiche, die aus einer Perspektive einer ersten Kamera verdeckt sind, von einer anderen Kamera erfasst werden.

In einem weiteren Ausführungsbeispiel kann die akustische Einheit Schall im hörbaren Bereich zeitaufgelöst erfassen. Allerdings kann es auch vorteilhaft sein, zusätzlich Töne und Geräusche im nicht-hörbaren Bereich zu erfassen, um eine besonders genaue Analyse der Tonentstehung und somit des davon abhängigen Therapieerfolgs zu ermöglichen. In einem bevorzugten Ausführungsbeispiel umfasst die akustische Einheit mindestens eine akustische Array-Kamera.

Vorzugsweise weist das System eine Ausgabeeinheit auf, um Vorgabedaten zu idealen Vorgängen, Trainingsanweisungen und/oder Analyseergebnisse anzuzeigen, die die Ausgabeeinheit beispielsweise von der Kontrolleinheit erhalten kann. Dabei kann die Ausgabeeinheit beispielsweise ein Gesicht, das die jeweilige Übung vorführt, und/oder das Gesicht des Benutzers während der Übung darstellen. Die Ausgabeeinheit kann Daten als Videofilm, als einzelne Bilder bzw. Töne oder eine Abfolge von mehreren Bildern bzw. Tönen akustisch und/oder optisch darstellen. Darüber hinaus kann die Ausgabeeinheit Überlagerungen von optischen und akustischen Daten und/oder Überlagerungen von erfassten und idealen Vorgängen ausgeben. Durch eine gleichzeitige Darstellung oder Überlagerung von Daten eines idealen abgespeicherten und eines vom Benutzer durchgeführten Vorgangs können Fehler in einem vom Benutzer durchgeführten Vorgang aufgezeigt bzw. eine ideale Durchführung verdeutlicht werden. Des Weiteren kann durch eine gleichzeitige Darstellung oder Überlagerung von akustischen und optischen Daten eine Korrelation zwischen optischen und akustischen Komponenten eines erfassten oder vorgegebenen Vorgangs veranschaulicht werden. Beispielsweise kann eine Lippenfehlstellung mit einer undeutlichen Artikulation bei Formung eines bestimmten Lauts einhergehen, was durch eine Überlagerung der optischen und akustischen Daten verdeutlicht wird.

Außerdem ist in einer bevorzugten Ausführungsform ein Speicher vorhanden, um Trainingsanweisungen, Analyseergebnisse, Daten über einen Trainingsverlauf, aufgenommene Vorgabedaten zu einem optimalen Vorgang und ähnliches zu speichern. Vorgabedaten von optimalen Vorgängen können entweder auf einer Computersimulation beruhen oder auf einer Vorführung des optimalen Sprech-, Atem-, Schluck- oder Inhalationsvorgangs durch einen Logopäden oder Therapeuten, der akustisch und optisch aufgezeichnet worden ist.

In einem bevorzugten Ausführungsbeispiel kann das System ferner eine Zeitlupenanalyse des erfassten Vorgangs durchführen. Dadurch kann ein Benutzer einen von ihm ausgeführten Vorgang oder auch einen vom System vorgeführten idealen Vorgang genau nachvollziehen. Insbesondere für eine überlagerte gleichzeitige Darstellung von akustischen und optischen Daten bzw. von erfassten und vorgegebenen Daten ist eine Ausgabe mit anpassbarer Geschwindigkeit vorteilhaft. Der Benutzer kann an kritischen Stellen die Ausgabe anhalten, verlangsamen oder wiederholen lassen, um diese besser nachvollziehen zu können.

In einem weiteren Ausführungsbeispiel kann das System eine Stimmhöhe, eine Lautstärke, eine Aussprache, eine Artikulation, ein Sprechtempo, eine Atmung, ein Inhalieren und/oder ein Schlucken analysieren. Dieses Ausführungsbeispiel ermöglicht dann nicht nur ein logopädisches Training bei neurologischen Insulten oder neurodegenerativen Erkrankungen (z.B. Schlaganfall, Parkinson), sondern auch ein Applikationstraining von Inhalatoren bei Asthma oder anderen Atemwegserkrankungen, eine Therapie bei Artikulationsstörungen, ein Aussprachetraining für Fremdsprachenschüler, eine Sprachanalyse im forensischen oder linguistischen Bereich, eine Optimierung von Behandlungsstrategien im Bereich der rekonstruktiven plastischen Chirurgie, Prothetik und Implantologie und eine stimmbildende Ausbildung von Schauspielern, Synchronsprechern und Sängern.

Bei einem erfindungsgemäßen Verfahren für Sprach- Schluck- und/oder Atemtraining werden zunächst dreidimensionale optische und ortsaufgelöste akustische Daten von einem trainierenden Patienten aufgenommen. Die erfassten Daten werden dann mit gespeicherten Daten verglichen und analysiert. Danach werden die Analyseergebnisse an den Patienten ausgegeben, um ihm ein Feedback zu liefern. Vorzugsweise erfolgt die akustische und optische Datenerfassung gleichzeitig und zeitaufgelöst.

### Kurze Beschreibung der Zeichnungen

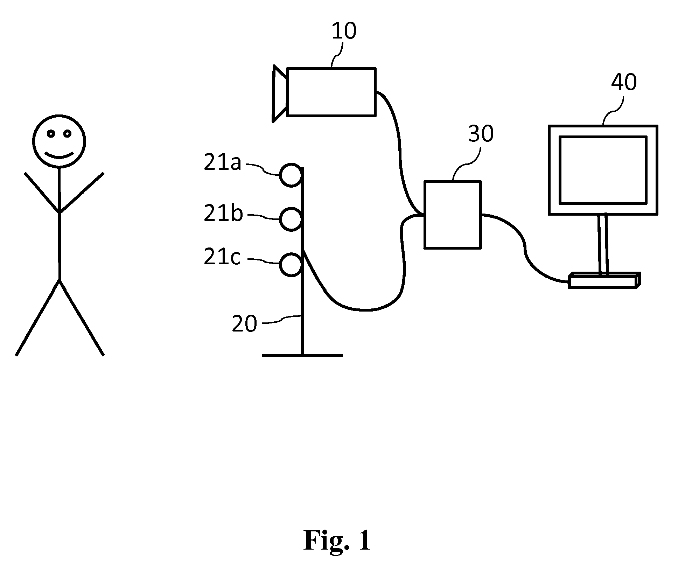

Figur 1 zeigt ein Ausführungsbeispiel eines erfindungsgemäßen Systems.
Figur 2 zeigt ein Ablaufdiagramm eines erfindungsgemäßen Verfahrens.

### Ausführliche Beschreibung der Erfindung

Das erfindungsgemäße System nimmt dreidimensionale optische und dreidimensionale akustische Daten in Echtzeit auf und kombiniert diese miteinander, um einen Trainingsvorgang möglichst genau zu erfassen. Hierfür umfasst das System eine optische Einheit 10 und eine akustische Einheit 20. Des Weiteren ist eine Kontrolleinheit 30 zur Analyse der erfassten Daten vorgesehen.

Die optische Einheit 10 kann ein beliebiges 3D-Kamerasystem umfassen, mit dem eine zeitaufgelöste dreidimensionale Bildaufnahme möglich ist, beispielsweise eine TOF-Kamera, eine Stereokamera, ein Triangulationskamerasystem oder ein interferometrisches System. Vorzugsweise wird allerdings mindestens eine TOF-Kamera eingesetzt, die ein dreidimensionales Bild basierend auf einer Signallaufzeit (time of flight) ermittelt. TOF-Kameras senden hierfür gepulstes und/oder moduliertes Licht aus, das von einem Objekt reflektiert wird. Die Entfernung zwischen Kamera und den einzelnen Punkten der Objektoberfläche kann entweder basierend auf der Signallaufzeit oder basierend auf einer reflektierten Photonenzahl ermittelt werden. TOF-Kameras verwenden beispielsweise einen PMD-Sensor (Photonenmischdetektor). Außerdem kann bei einer TOF-Kamera Licht im nahen Infrarotbereich verwendet werden, das nicht gesundheitsschädlich ist. Als Lichtquellen kommen beispielsweise LEDs oder Laserdioden in Frage. Darüber hinaus kann Fremdlicht effizient unterdrückt werden, indem das aktive Sendersignal aus dem Umgebungslicht herausgefiltert wird. Dadurch wird auch ein Einsatz unter schwierigen Umgebungsbedingungen ohne Kalibrierungen möglich. Auf diese Weise ist eine Echtzeit-Erfassung einer dreidimensionalen Oberfläche möglich. Zur Unterscheidung eines Benutzers von einem Hintergrund kann ein Entfernungs-Offset vorgegeben werden, wobei nur Bildpunkte mit kürzerer Entfernung berücksichtigt und alle anderen Bildpunkte als Hintergrund betrachtet werden. Vorzugsweise werden mindestens zwei Kameras eingesetzt, um einen echten dreidimensionalen Datensatz zu generieren. Insbesondere bei der Verwendung des Systems zum Trainieren einer Inhalatorapplikation ist es vorteilhaft eine weitere Kamera zu verwenden, da ein Teil des Mundes von der Hand des Übenden, die den Inhalator hält, verdeckt wird. Dies kann durch seitliches Anbringen einer zweiten oder dritten Kamera ausgeglichen werden.

Die akustische Einheit 20 kann eine akustische Array-Kamera umfassen, bei der mehrere Mikrofone 21a, 21b und 21c in einer geeigneten Geometrie angeordnet sein können. Die verschiedenen Mikrofone 21a, 21b und 21c sind durch Laufzeitkorrektur auf verschiedene Messpunkte fokussiert, sodass ein Bild ortsselektiv abgehört werden kann. Möglicherweise umfasst die akustische Array-Kamera außerdem eine Videokamera, um den gemessenen Schall von verschiedenen Messpunkten Bildpunkten eines Videobilds zuzuordnen. Die Auflösung einer akustischen Array-Kamera kann beispielsweise 1 mm³ für die Ortsauflösung bzw. 5 Hz für die Frequenzauflösung betragen. Vorzugsweise kann Schall im gesamten hörbaren Bereich erfasst werden, d. h. von ca. 15 Hz bis ca. 20 KHz mit einem Pegel von etwa 10 dB. Vorzugsweise kann die akustische Einheit 20 unterschiedliche Orte der Schallentstehung erfassen und diese nach Frequenz, Amplitude und Dauer auflösen, sodass z.B. ein Spektrum eines Ortes, eine Spektrum eines Zeitintervalls, ein Spektrum eines Frequenzintervalls oder der Klang eines Ortes aufgenommen werden kann. Ebenfalls denkbar sind 3D-Schall(druck)-Messungen mit anderen als herkömmlichen massegebundenen Mikrofonen, also z.B. reinen Druckmessern nach Pirani, Venturi, Thermocoupling, Laser-Doppler-Anemometrie (LDA) oder DMS-Sensor-Prinzip. Bei diesen Lösungen bestünde der Vorteil der nahezu masselosen und daher trägheitslosen schnelleren Messung. Das ist wichtig wegen der Realtime-Verarbeitung der Daten.

Die Kontrolleinheit 30 für die Analyse eines Trainingsvorgangs basierend auf den akustischen und optischen Daten umfasst beispielsweise einen Computer. Die Kontrolleinheit 30 vergleicht dabei vorzugsweise die erfassten Daten mit vorgegebenen Daten, die in einem Speicher gespeichert sein können. Diese vorgegebenen Daten bzw. Vorgabedaten können auf einer Computersimulation oder auf Daten basieren, die bei einer Vorführung durch einen Therapeuten optisch und/oder akustisch aufgenommen worden sind. Entsprechend der Analyse gibt die Kontrolleinheit 30 dem Benutzer akustische und/oder optische Trainingsanweisungen über eine Ausgabeeinheit 40 aus, die vorzugsweise mindestens einen Bildschirm und/oder einen Lautsprecher umfasst.

Beispielsweise die Ausgabeeinheit 40 ein Gesicht oder eine Person anzeigen, die die gewählte Übung vorführt. Zusätzlich kann auch der Benutzer dargestellt werden. In einem Ausführungsbeispiel kann der Benutzer eine vorführende Person, die ihm am ehesten entspricht, von mehreren Personen auswählen. Ein Kind kann demnach ein Kind als vorführende Person wählen, was bezüglich der Kopfgeometrie und Größe besser entspricht. Alternativ können auf der Ausgabeeinheit 40 Graphen dargestellt werden, in denen eine Zielvorgabe und das vom Benutzer erzielte Ergebnis angezeigt werden. Solche Graphen können Frequenz-Intensität-Darstellungen, Frequenz-Zeit-Darstellungen, Intensität-Zeit-Darstellungen u.ä. sein. Außerdem kann die Ausgabeeinheit 40 eine Überlagerung der erfassten optischen und akustischen Daten zeitaufgelöst, etwa als Video, ausgeben, wobei beispielsweise die dreidimensionale Körperoberfläche durch Graustufen und die Intensität oder Frequenz von Tönen am Ort der Tonentstehung farbkodiert dargestellt werden. Auf diese Weise kann ein Benutzer leicht erkennen, welche Bewegungen er beim Training durchführt und an welchem Ort welche Töne entstehen. Zusätzlich zur Darstellung der Performance des Benutzers kann ferner ein optimaler Vorgang dargestellt werden. Hierbei können zusätzlich optische und akustische Daten, die einen optimalen Vorgang simulieren, in Graustufen bzw. farbkodiert dargestellt werden, sodass der Benutzer sein erzieltes Ergebnis mit der Vorgabe vergleichen kann. Des Weiteren können die Analyseergebnisse der erfassten Daten ausgegeben werden, indem beispielsweise zu stark oder zu wenig benutzte Muskelgruppen oder Gesichtspartien mit Pfeilen oder Farbe angezeigt werden. Eine zu hohe bzw. zu niedrige Stimme, eine zu große bzw. zu geringe Lautstärke oder ein zu schnelles bzw. zu langsames Sprechtempo kann mit nach unten bzw. nach oben gerichteten Pfeilen korrigiert werden. Analog dazu kann ein Schluck- oder Atmungsprozess analysiert und trainiert werden. Die Trainingsanweisungen können auch akustisch ausgegeben werden, vorzugsweise aber werden die Trainingsanweisungen akustisch und optisch ausgegeben. Durch die Erfassung sämtlicher für das Training relevanter Parameter kann dem Benutzer ein sehr genaues Feedback gegeben und schon kleine Trainingserfolge erfasst werden. Vorzugsweise kann der Benutzer auch einen Trainingsverlauf abfragen, evt. aufgeschlüsselt nach den verschiedenen Trainingsparametern wie Lautstärke, Stimmhöhe, etc., der basierend auf zuvor erzielten Trainingswerten ermittelt wird. Beispielsweise kann eine Sprechübung das Sprechen mit lauter Stimme trainieren, wobei der Benutzer zu immer lauterem Sprechen angeregt wird. Auf ähnliche Weise kann eine Stimmhöhe, Lautstärke, Aussprache, Artikulation, Mimik, Atmung, eine Atmungseinteilung beim Sprechen, ein Sprechtempo, Schlucken, usw. korrigiert werden. Vorzugsweise ist das System auch zum Trainieren eines Inhalationsvorgangs insbesondere mit nicht-Atemzug gesteuerten Inhalatoren geeignet oder kann für die Entwicklung von Inhalatoren verwendet werden.

Um einen Vorgang, z. B. Sprechen, Schlucken, Atmen, Inhalieren etc., mittels einem erfindungsgemäßen Verfahren zu trainieren bzw. zu bewerten, werden zunächst akustische und optische dreidimensionale Daten mittels der akustischen Einheit 20 und der optischen Einheit 10 aufgenommen und an die Kontrolleinheit 30 weitergegeben. Die Kontrolleinheit 30 analysiert die empfangenen Daten, indem sie die Daten beispielsweise mit abgespeicherten Vorgabedaten vergleicht. Entsprechend des Analyseergebnisses werden dann über die Ausgabeeinheit 40 Analyseergebnisse und/oder Trainingsanweisungen optisch und/oder akustisch ausgegeben. Auf diese Weise erhält der Benutzer ein genaues und unmittelbares Feedback zu seinem Training.

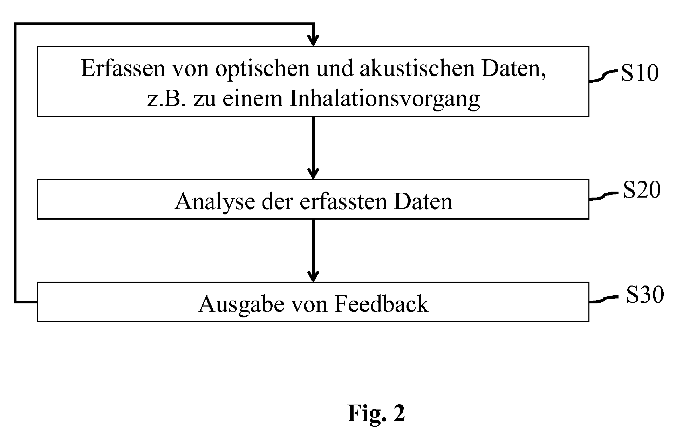

Ein Ablauf des erfindungsgemäßen Verfahrens ist in Figur 2 dargestellt. Während ein Benutzer eine Übung zum Trainieren eines Sprech-, Schluck-, Atmungs- oder Inhalationsvorgangs ausübt, werden in Schritt S10 die Durchführung der Übung optisch und akustisch in Echtzeit aufgenommen. Alternativ oder zusätzlich können optische und akustische Daten auch gleichzeitig erfasst werden. Die erfassten Daten werden in Schritt S20 beispielsweise durch Vergleichen mit Vorgabedaten oder durch optische und/oder akustische Mustererkennung analysiert. Die Analyse der optischen und akustischen Daten kann gleichzeitig oder nacheinander erfolgen. Möglicherweise wird die akustische Datenanalyse unabhängig von der optischen Datenanalyse durchgeführt. Es ist allerdings vorzuziehen, zumindest bis zu einer gewissen Stufe der Analyse die Auswertung der akustischen und optischen Daten abhängig von einander durchzuführen. Wenn in diesem Fall beispielsweise basierend auf den möglicherweise schneller ausgewerteten akustischen Daten deutlich wird, dass die Übung fehlgeschlagen ist oder nicht ausgewertet werden kann, kann die Auswertung der akustischen sowie der optischen Daten abgebrochen werden. Die Analyseergebnisse und möglicherweise auch die Rohdaten können gespeichert werden, um später für eine Anzeige des Trainingsverlaufs o.ä. zur Verfügung zu stehen. Entsprechend der Analyse wird in Schritt S30 dem Benutzer akustisches und/oder optisches Feedback gegeben. Beispielsweise kann bei erfolgreicher Durchführung der Übung ein positives Feedback oder bei verbesserungswürdiger Durchführung ein korrigierendes Feedback gegeben werden. Darüber hinaus kann auch eine Fehlermeldung oder eine Aufforderung zur Wiederholung der Übung ausgegeben werden.

Im Folgenden wird der Trainingsvorgang beispielhaft anhand eines Inhalationstrainings beschrieben. Die Anwendung von Wirkstoffen in Form inhalierbarer Aerosole zur Behandlung von entzündlichen oder obstruktiven Atemwegserkrankungen, wie zum Beispiel Asthma bronchiale oder COPD, sind seit langem bekannt. Als Wirkstoffe zur Behandlung der Asthma bronchiale kommen üblicherweise Glucocorticoide, wie beispielsweise Budesonid, Ciclesonid, Fluticason, Beclometason oder Mometason; Beta-2-Sympathomimetika, beispielsweise Formoterol, Salmeterol, Fenoterol oder Indacaterol; oder Anticholinergika, beispielsweise Ipratropium, Glycopyrrolat oder Tiotropiumbromid, zum Einsatz. Um einen optimalen Inhalationsvorgang sicherzustellen, der für einen optimalen Therapieerfolg mit Aerosolen von grundlegender Bedeutung ist, kann ein Benutzer mit Hilfe des erfindungsgemäßen Systems bzw. Verfahrens die korrekte Handhabung eines Inhalators trainieren bzw. überwachen. Hierfür inhaliert ein Benutzer mit dem Inhalator, wobei dieser Vorgang optisch und akustisch in Schritt S10 aufgezeichnet wird. Bei der Auswertung der aufgenommenen Daten in Schritt S20 kann dabei besonderes Augenmerk darauf gelegt werden, dass der Mund des Benutzers das Mundstück gut umschließt, dass der Inhalator richtig gehalten wird oder dass der Benutzer tief genug einatmet. Dies kann anhand des Entstehungsortes von Geräuschen, Geräuschpegel, Mustererkennung und Ausmaß der Brustkorbbewegung erfolgen. Für eine Mustererkennung ist es zudem vorteilhaft, wenn ein Inhalator mit bekannter Form verwendet wird, um zwischen Inhalatormundstück und Gesicht des Benutzers unterscheiden bzw. die Stellung des Inhalators erkennen zu können. In Schritt S30 können dann dem Benutzer erfasste Inhalationsvorgänge, optimale Inhalationsvorgänge, Analyseergebnisse und/oder Anweisungen zum Ausführen der Übung, etc. angezeigt werden. Die Analyseergebnisse können auch eine Bewertung des durchgeführten Inhalationsvorgangs beinhalten und dem Benutzer beispielsweise einen erfolgreichen bzw. einen nicht-erfolgreichen, zu wiederholenden Inhalationsvorgang anzeigen. Die erfassten Daten und/oder die Analyseergebnisse können zudem an einen betreuenden Mediziner oder Therapeuten gesendet werden, sodass auch eine persönliche medizinische Überwachung per Telemedizin ermöglicht wird.

Das erfindungsgemäße System und Verfahren bietet eine günstige und individuell abgestimmte Trainingsmöglichkeit, die ohne großen Aufwand (Anfahrt) betrieben werden kann und dem Benutzer permanent Feedback zu kleinsten Fortschritten geben kann. Somit kann ein sicherer Trainingsweg aufgezeichnet werden und der Fortschritt durch Compliance sichtbar gemacht werden. Auch kann das erfindungsgemäße System bzw. Verfahren im Bereich der Telemedizin oder Teletherapie eingesetzt werden, wobei der vom Benutzer ausgeführte Vorgang von einem betreuenden Mediziner oder Therapeuten beurteilt werden kann. Außerdem können Komplikationskosten eingespart werden, die durch Folgeerkrankungen, wie Bronchitis oder Lungenentzündung bei zu flacher Atmung, entstehen können. Ferner kann die Krankheitsprogression beispielsweise bei Parkinson verlangsamt werden und Patienten erhalten durch verbesserte Sprache mehr Autonomie und Lebensqualität. Darüber hinaus wird die Leistung der logopädischen Therapie messbar gemacht.

## Patentansprüche

1. System zum Analysieren und/oder Trainieren eines Sprach-, Schluck- und/oder Atemvorgangs, umfassend:
eine optische Einheit (10) zur dreidimensionalen optischen Datenerfassung;
eine akustische Einheit (20) zur ortsaufgelösten akustischen Datenerfassung; und
eine Kontrolleinheit (30), die dazu geeignet ist, einen Sprech-, Schluck- und/oder Atemvorgang basierend auf den akustischen und optischen Daten zu analysieren.

2. System nach Anspruch 1, wobei die Kontrolleinheit (30) die akustischen und optischen Daten durch einen Vergleich mit Vorgabedaten analysiert.

3. System nach Anspruch 2, wobei die Vorgabedaten Computersimulationsdaten und/oder Daten umfassen, die bei einer Vorführung eines jeweiligen optimalen Vorgangs durch eine geschulte Person erfasst worden sind.

4. System nach einem der obigen Ansprüche, wobei das System ferner umfasst:
einen Speicher zum Speichern von Vorgabedaten, Trainingsanweisungen, Analyseergebnissen und/oder Daten eines Trainingsverlaufs; und/oder
eine Ausgabeeinheit (40), die Vorgabedaten, Trainingsanweisungen, Analyseergebnisse, Überlagerungen der erfassten optischen und akustischen Daten und/oder Überlagerungen von erfassten und vorgegebenen Daten akustisch und/oder optisch ausgibt.

5. System nach einem der obigen Ansprüche, wobei das System dazu geeignet ist, die akustischen und optischen Daten in Echtzeit zu erfassen und/oder einen erfassten Vorgang in Zeitlupe zu analysieren.

6. System nach einem der obigen Ansprüche, wobei das System dazu geeignet ist, Stimmhöhe, Lautstärke, Aussprache, Artikulation, Sprechtempo, Atmung, Schlucken und/oder einen Inhalationsvorgang zu analysieren.

7. System nach einem der obigen Ansprüche, wobei die optische Einheit (10) dazu geeignet ist, Bewegung einer Körperoberfläche und/oder Anspannung von Muskeln aufzulösen.

8. System nach einem der obigen Ansprüche, wobei die akustische Einheit (20) dazu geeignet ist, Schall bezüglich Frequenz, Amplitude, Dauer und/oder Intensität in Echtzeit aufzulösen und/oder verschiedene Orte von Schallentstehung zu erfassen.

9. System nach einem der obigen Ansprüche, wobei die optische Einheit (10) mindestens eine TOF-Kamera und/oder die akustische Einheit (20) mindestens eine akustische Kamera umfasst.

10. Verwendung eines Systems nach einem der Ansprüche 1-9 zum Trainieren einer Inhalatorapplikation zur Behandlung von entzündlichen und/oder obstruktiven Atemwegserkrankungen.

11. Verfahren zum Analysieren und/oder Trainieren eines Sprach-, Schluck- und/oder Atemvorgangs, die folgenden Schritte umfassend:
Erfassen (S10) von dreidimensionalen optischen und ortsaufgelösten akustischen Daten;
Analyse (S20) eines Sprech-, Schluck- und/oder Atemvorgangs basierend auf den akustischen und optischen Daten; und
Ausgabe (S30) der Analyseergebnisse.
